# DEMANDE DE BREVET EUROPEEN

(11) **EP 1 707 121 A1**
(43) Date de publication de la demande: **04.10.2006**
(21) Numéro de dépôt: 05447070.3
(22) Date de dépôt: 30.03.2005
(51) Int. Cl.: A61B 5/11, A61B 5/04, A61N 1/36

(54) **Dispositif de mesure de l'activité rythmique de fibres musculaires**

(71) Demandeur: UNIVERSITE LIBRE DE BRUXELLES, 1050 Bruxelles (BE)
(72) Inventeur: Manto, Mario, 1440 Braine-le-Château (BE)
(74) Mandataire: pronovem

(57) **Abrégé**

La présente invention se rapporte à un dispositif portatif de mesure des fréquences de tremblements musculaires d'un sujet, comportant des électrodes de mesure dites actives (2) aptes à pénétrer la peau et/ou le muscle du sujet, connectés à un micro-contrôleur (5) collectant les courants électriques mesurés par les électrodes et affichant instantanément la fréquence correspondante dudit tremblement du sujet sur un écran (6).

## Description

### Objet de l'invention

La présente invention est relative à un dispositif permettant directement une mesure de la fréquence d'un tremblement musculaire d'un sujet, permettant d'améliorer le diagnostic des pathologies dont souffre le sujet et éventuellement d'améliorer le suivi thérapeutique de ce sujet.

### Arrière-plan technologique et état de la technique à la base de l'invention

Les tremblements sont définis comme un mouvement musculaire rythmique involontaire, présentant une fréquence ou une gamme de fréquences définies généralement entre 2 Hz et 18 Hz. Les tremblements chez les sujets humains constituent l'une des pathologies neurologiques les plus fréquentes. Les études récentes estiment que 3 à 4 % de la population humaine de plus de 60 ans souffriraient de tremblements des membres et/ou de la tête. Actuellement, l'analyse des tremblements en milieu hospitalier fait appel à une combinaison d'appareils cinématiques (goniométres, gyroscopes, accéléromètres, vidéomètres) et électromyographiques. Ces enregistrements nécessitent plusieurs appareils et requièrent un traitement complexe des données acquises par ces appareils de mesure.

En outre, on ne dispose pas d'un outil qui permette d'identifier si deux fibres musculaires présentant des activités rythmiques sont contrôlées par le même motoneurone et de connaître de manière simultanée la fréquence de tremblement de l'ensemble de ces fibres musculaires. Ceci est particulièrement gênant lorsqu'il s'agit de suivre des patients ayant subi une lésion d'un nerf périphérique. Dans ce cas en effet, il peut y avoir chez le patient une réorganisation des jonctions musculaires et/ou l'apparition d'un tremblement (Proudlock FA, Scott J. Brain Res. 2003 Nov. 7 ; 989 (2) : 238 -45 ; Nobrega JC (Arq Neuropsiquiatr. 2002 Mar ;60 (1) : 17-20 ; Milanov I. Electromyogr. Clin. Neurophysiol. 2001 Dec ; 41 (8) : 479-84), ce qui peut aboutir à ce que, après reconstitution des fibres musculaires, des fibres musculaires qui étaient initialement innervées par le même motoneurone ne le sont plus.

### Buts de l'invention

La présente invention vise à fournir un dispositif simple, portatif, non invasif, et ergonomique qui permette une mesure instantanée, par un personnel médical, de la fréquence de tremblement d'un sujet, de préférence un patient humain, et également une mesure plus spécifique de l'activité rythmique des différentes fibres musculaires permettant d'assurer le suivi thérapeutique des bouffées et des contractions musculaires chez certains sujets.

La présente invention a aussi pour but d'améliorer le diagnostic neurologique des fréquences de tremblements, directement au lit du sujet (patient humain malade) ou lors de consultations générales de neurologie.

En outre, la présente invention a pour but de fournir un diagnostic direct et instantané des tremblements les plus fréquents chez un sujet, en particulier le tremblement orthostatique.

### Eléments caractéristiques de l'invention

La présente invention concerne un dispositif de préférence portatif, non invasif et ergonomique, comprenant des électrodes (de mesure), de préférence des électrodes dites actives, aptes à pénétrer la peau et/ou le muscle du sujet; lesdites électrodes de mesure étant reliées à un micro-contrôleur collectant la fréquence mesurée par les électrodes d'un tremblement éventuel d'un sujet et affichant la fréquence du tremblement du sujet enregistré sur un écran, de préférence de type STN.

Dans le dispositif de l'invention, le signal des électrodes est donc amplifié, filtré et redressé pour obtenir un signal analogique transformé en signal digital et traité par un microcontrôleur de type PIC de Microchip (de préférence un microcontrôleur PIC 18F ou dsPIC) (digital signal controler, tel que le dsPIC 30F) qui collecte et affiche la fréquence du tremblement mesuré.

De préférence, le dispositif comprend également une surface autocollante apte à permettre l'application des électrodes dans la peau et/ou dans le muscle du sujet.

De préférence, le dispositif permet la mesure des fréquences de tremblement de sujets dont les fréquences sont comprises entre environ 1 Hz et environ 25 Hz, de préférence entre environ 2 Hz et environ 18 Hz, plus particulièrement permettant la mesure du tremblement orthostatique dont la fréquence est comprise entre environ 13 Hz et environ 17 Hz.

Le dispositif de l'invention peut également être relié à d'autres unités d'affichage de la fréquence du tremblement, tel qu'un écran d'ordinateur, de préférence un ordinateur portable, relié à des micro-électrodes d'enregistrement de l'activité d'un muscle ou à des micro-électrodes de stimulation de nerfs liées à un motoneurone; ces micro-électrodes d'enregistrement ou de stimulation de nerfs étant connectées au micro-contrôleur du dispositif de l'invention pour collecter des mesures permettant de caractériser une innervation individuelle de fibres musculaires par un moto-neurone.

### Description détaillée de l'invention

Le dispositif de mesure d'activités rythmiques de fibres musculaires de l'invention est un appareillage simple, portable et ergonomique qui permet de détecter instantanément une fréquence d'un tremblement affectant un sujet, de préférence un patient humain.

Le dispositif de l'invention peut être éventuellement combiné à (ou présent dans) d'autres outils de diagnostic qui sont les outils habituels d'un médecin, à savoir : des stéthoscopes, en particulier des stéthoscopes électroniques actuellement commercialisés.

En outre, ce dispositif peut être éventuellement combiné à d'autres appareillages basés sur les techniques cinématiques et électromyographiques, de manière à perfectionner le diagnostic ou le suivi médical d'un sujet, de préférence un patient humain souffrant de tremblements.

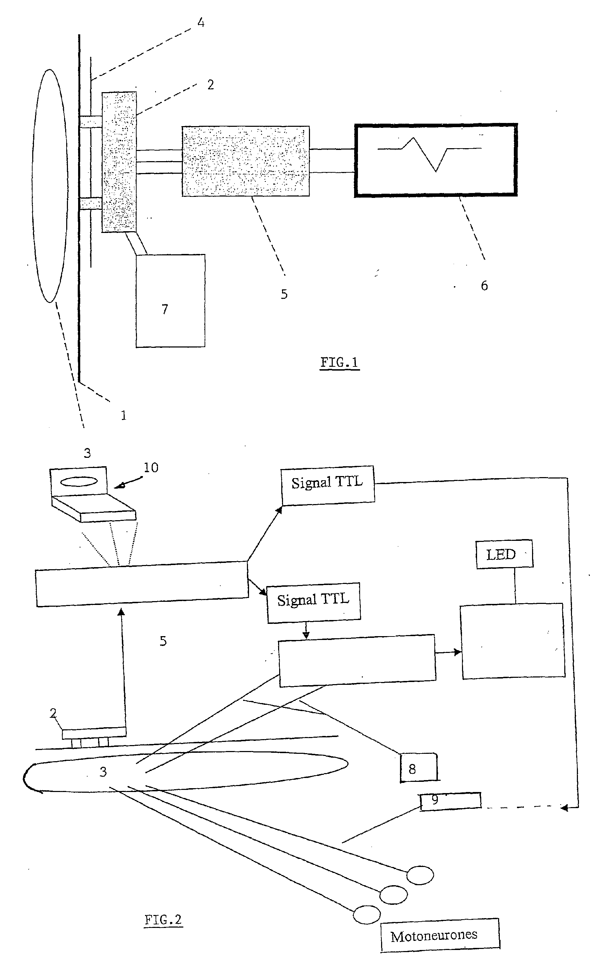

Tel que représenté à la figure 1, le dispositif de l'invention présente une configuration simple permettant de mesurer instantanément la fréquence d'un tremblement musculaire en appliquant directement le dispositif de l'invention sur la peau (1) du sujet et par une mise en contact de plusieurs électrodes de mesure (2) avec la peau (1) et/ou le muscle (3) du sujet. Ce contact peut éventuellement être fait par l'intermédiaire d'une surface collante (4) assurant un contact constant et efficace entre les électrodes de mesure (2), la peau (1) et/ou le muscle (3) du sujet (afin d'obtenir une mesure efficace (en réduisant les perturbations induites par un bruit de fond) de la fréquence du tremblement). Les électrodes de mesure (2) sont des électrodes dites de « surface avec pré-amplification », qui permettent un gain de mille fois par rapport à une électrode classique, ces électrodes de mesure (telles que les électrodes Delsys 2.3) sont dites « actives ». En outre, ces électrodes de mesure peuvent comporter des filtres intégrés (« low pass filter » et « high pass filter ») permettant de filtrer les signaux de fréquence parasite de manière à assurer avantageusement une mesure efficace de la fréquence du tremblement sans être gêné par un bruit de fond.

Le dispositif de l'invention comporte en outre un micro-contrôleur (5) (apte à collecter en fonction du temps les signaux électriques mesurés par les électrodes de mesure, à les traiter, et à calculer la fréquence de l'activité musculaire associée), un écran (affichant instantanément cette fréquence pour le personnel soignant), plus particulièrement un écran de type STN (6) et un système d'alimentation (telle qu'une pile (7)), de manière à rendre le système de l'invention portable et autonome (sans connexion extérieure(wireiess))

Les différents éléments interagissent entre eux via des connexions (de préférence fixes) ou sans fil de connexion (wireless). Une connexion sans fil existe entre le micro-contrôleur (5) et l'écran (6) qui est dans ce cas un écran d'un dispositif électronique indépendant (entre les mains du personnel hospitalier) (tel qu'un écran apparaissant sur un bracelet-montre, un écran d'un ordinateur (10), un écran d'un cahier de notes (notebook), etc.

De même, le dispositif peut également comporter des systèmes d'alimentation indépendants autres que des piles, par exemple un écran solaire.

Par le dispositif de l'invention, le signal électrique provenant des électrodes (de mesure) dites actives (2) est amplifié et filtré et ensuite collecté et traité par le micro-contrôleur (5) qui transmet instantanément les données de mesure (c'est-à-dire la valeur de la fréquence mesurée) sur un écran (6) qui offre un affichage instantané de la fréquence d'oscillation (valeur numérique ou graphique) et éventuellement l'enveloppe du signal, c'est-à-dire l'évaluation du signal en fonction du temps.

Le dispositif de l'invention peut être posé de manière permanente sur le sujet (patient humain) via des systèmes de connexion permettant un affichage de la fréquence du tremblement sur des vêtements formant écran (par exemple la ceinture du patient). Ces systèmes de connexions peuvent éventuellement être incorporés directement dans les vêtements du sujet (dans le cas d'un patient humain).

De même, le dispositif de l'invention peut être utilisé pour analyser le comportement des bouffées musculaires lors de tremblements humains avec un affichage direct de la fréquence dominante et d'autres mesures de l'activité musculaire, en particulier : le pic d'amplitude, la moyenne du signal redressé, la période réfractaire après une bouffée, le rapport du pic maximal sur l'intégrale de la bouffée d'amplitude la plus faible (ce rapport est un indicateur du degré de synchronisation des décharges des motoneurones), la modulation de l'amplitude des bouffées que l'on estime par un écart-type des maximums des bouffées successives sur une période de temps prédéterminée et le « crest-factor » pour chacune des bouffées successives (rapport des pics par intégrales pour chaque bouffée).

[0021] Le dispositif permet également d'évaluer de manière précise la fréquence et le type de tremblements humains et de préférence préciser les effets des traitements médicaux et chirurgicaux en cours de rééducation neurologique.

En outre, le dispositif de l'invention permet un affichage instantané des fréquences typiques du tremblement parkinsonien ou du tremblement essentiel lors des procédures de neurostimulation cérébrale en salle d'opération.

Ceci permet d'améliorer aussi la procédure de localisation des noyaux cérébraux (noyau sous-thalamique et noyau VIM), ainsi que le diagnostic direct du tremblement orthostatique directement dans un cabinet de consultations par un médecin généraliste ou un neurologue.

Le dispositif de l'invention est muni d'une alarme sonore qui se déclenche automatiquement dès qu'un tremblement de fréquence 14 à 18 Hz (tremblement orthostatique) est détecté. Il s'agit ainsi du premier outil de dépistage de cette pathologie utilisable par du personnel « non » ou « peu » qualifié, ou le patient lui-même. Les laboratoires qui pratiquent ce type d'analyse utilisent un matériel EMG encombrant et qui nécessite du personnel qualifié pour analyser les données collectées.

Grâce à cette alarme, le dispositif permet au personnel soignant ou au patient qui souffre d'un tremblement de type « high-frequency synchronous discharges » (décharges musculaires de 14 à 18 Hz au niveau des bras) de confirmer l'apparition de ce tremblement (qui a un caractère explosif) et de le bloquer en réalisant des extensions forcées du poignet (référence : Arch. Neurol. 2003 ;60 : 416-422). Ces décharges explosives de haute fréquence sont observées chez les patients qui présentent une atrophie olivopontocérébelleuse. Grâce au dispositif de l'invention, on peut explorer pour la première fois avec un outil qui tient dans une main la période réfractaire après une bouffée, par le calcul instantané de l'effet synchronisateur d'un stimulus externe. Ce calcul est réalisé sur une différence entre des bouffées mesurées et des bouffées prédites. Dans les « high-frequency synchronous discharges » il y a un haut effet de re-synchronisation post-stimulus.

Le tremblement orthostatique humain présente de manière spécifique une fréquence comprise entre environ 13 et environ 17 Hz. L'affichage instantané de la valeur d'une telle fréquence permet d'identifier rapidement la pathologie dont souffre le patient. Le dispositif peut également comprendre un moyen d'affichage spécifique (lumineux) ou d'alarme (sonore) lorsque ce type de tremblement est mesuré.

En outre, le dispositif de l'invention permet également un diagnostic instantané du tremblement postural des membres inférieurs à 3 Hz chez les sujets présentant une atrophie du lobe antérieur du cervelet d'origine alcoolique et peut être également impliqué dans le suivi d'études pharmacologiques, en particulier l'évaluation directe des effets de nouveaux composés sur le tremblement parkinsonien ou le tremblement essentiel notamment.

Le dispositif de l'invention peut être également plus complexe et combiné à un certain nombre d'unités indépendantes qui forment un système qui permet une détection et éventuellement une analyse simultanée par une multitude d'électrodes de la réaction de plusieurs groupes musculaires. Un groupe musculaire est défini comme au minimum deux muscles contigus. Ce système permet de détecter et éventuellement d'analyser des différences de fréquences de décharges entre les différents muscles, par exemple, des muscles de différents membres d'un même sujet.

Le dispositif de la présente invention peut être également combiné à d'autres éléments pour détecter de manière directe la fréquence d'un tremblement et simultanément déterminer si deux fibres musculaires dépendant d'un même muscle (faisant l'objet d'une même activité rythmique) sont innervées par le même motoneurone.

Par conséquent, le dispositif ou système de l'invention peut trouver également des applications dans le domaine de la recherche, mais également dans le domaine du diagnostic de lésion de nerfs périphériques d'un sujet (en particulier d'un patient humain).

Le dispositif de l'invention vise en particulier à analyser le synchronisme de décharge de deux fibres d'un sujet (en particulier d'un patient humain) soumises à une activité rythmique. L'activité de chaque fibre est détectée par une micro-électrode implantée dans les muscles au travers de la peau du sujet, le dispositif de l'invention générant ensuite un signal sur un écran, de préférence une LED qui s'allume lorsque le dispositif détecte que deux fibres ne sont pas innervées par le même motoneurone.

Dans ce cas, le dispositif de l'invention comportera, tel que représenté à la figure 2, des électrodes actives (2) en contact direct avec le muscle (3) du sujet ; ces électrodes étant éventuellement maintenues sur la peau (1) du patient via une interface collante (4). Les électrodes de mesure dites actives (2) sont directement connectées à un micro-contrôleur (5) permettant un traitement de la mesure FFT (mesure de la transformée de Fourier) c'est-à-dire un traitement et un affichage de la fréquence du tremblement mesuré par les électrodes qui pourra être transmise par connexion directe ou par transmission infrarouge (IR) (wireless) sur un écran, éventuellement sur un écran d'ordinateur (10).

En outre, le micro-contrôleur (5) permettra un traitement du signal TTL (le TTL correspond à une porte logique de 0 à volts (signal rectangulaire non alternatif) de la mesure et une comparaison avec les mesures provenant de microélectrodes d'enregistrement (8) connectées directement au muscle du sujet et provenant des micro-électrodes de stimulation de nerfs (9) connectées directement au motoneurone du sujet. Le microcontrôleur (5) permet dans ce cas une amplification des mesures (signaux mesurés) entre ces différentes électrodes et une conversion de l'analyse du signal par l'intermédiaire éventuel d'une LED qui s'allume lorsque deux fibres ne sont pas innervées par le même motoneurone.

Suite au résultat de la mesure du signal TTL provenant du microcontrôleur (5) une stimulation du nerf durant les périodes de tremblement est provoquée afin de mesurer un éventuel phénomène de synchronisation par stimulation électrique.

Ces différentes mesures sont faites selon le protocole présenté ci-dessous.

### Amplification du signal

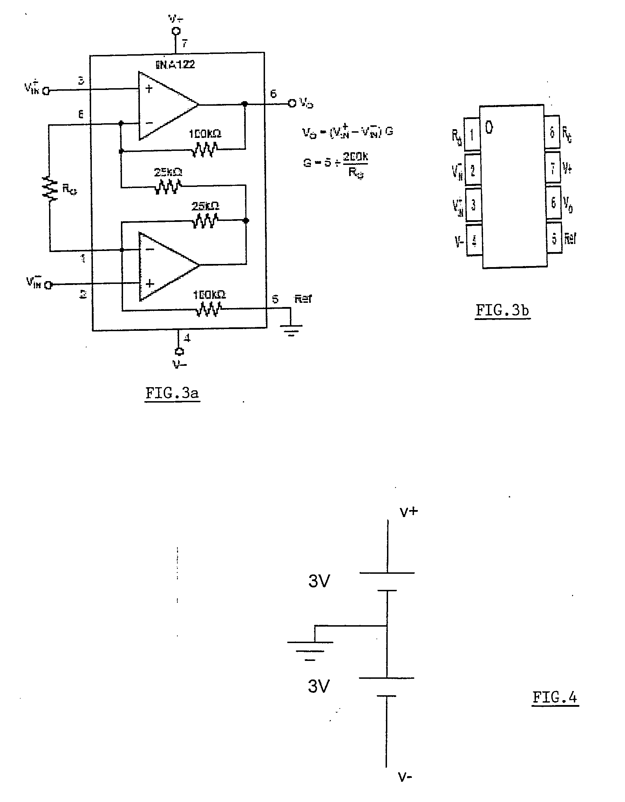

L'amplification du signal de mesure issu de fibres musculaires isolées est obtenue par un ampli d'instrumentation conçu pour des applications en électromyographie (EMG), en particulier le circuit INA122™ single supply micro power instrumentation amplifier (Texas Instruments) (voir figures 3a et 3b) .

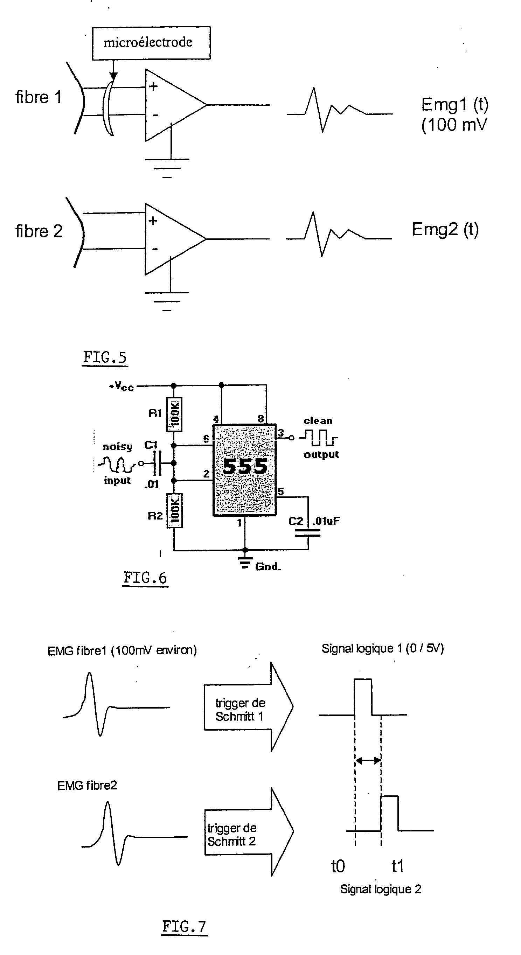

Le gain G de l'amplificateur est variable et est fixé par la résistance Rg. On choisit Rg de manière à avoir un gain de 100 environ. On alimente l'amplificateur à l'aide d'une alimentation symétrique de +/- 3V (figures 4 et 5) .

### Conversion du signal électromyographique (EMG) et impulsions

On analyse le synchronisme des signaux des mesures provenant des deux fibres, après conversion des signaux analogiques en signaux logiques plus facilement exploitables (on utilise pour cela un trigger de Schmitt construit avec un oscillateur Ne 555) (figure 6) dont les résistances R1 et R2 doivent être ajustées en fonction du seuil à détecter sur le signal EMG. Sur base de l'activité enregistrée au niveau des fibres musculaires, ce circuit génère une impulsion logique de 5V pour chaque activité EMG mesurée. Par le déphasage entre ces deux signaux logiques, on déduit un synchronisme de décharge des fibres musculaires (voir figure 7).

### Création du circuit d'analyse des signaux logiques

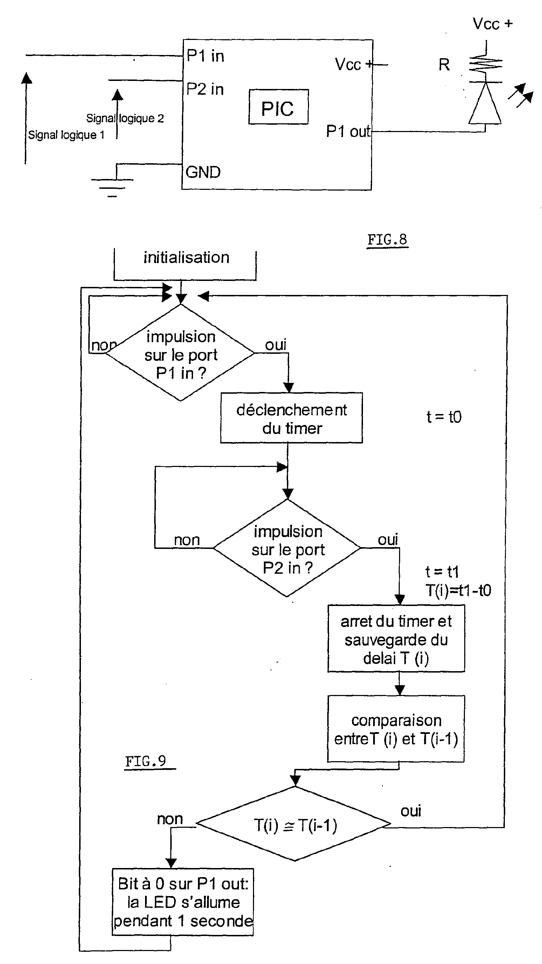

On utilise un microcontrôleur de type PIC (de Microchip) (PIC 18F) programmable en Basic. Il s'agit d'un composant simple (contenant des ports d'entrée-sortie, un timer interne, une unité arithmétique et logique, une RAM interne et quelques registres) qui convient pour des applications ne nécessitant pas une grande puissance de calcul pour sa taille réduite, sa faible consommation et son faible coût. En outre, on utilise également un microprocesseur, une carte de programmation et un logiciel d'interface avec l'ordinateur (PC). On utilise deux ports d'entrée pour signaux logiques (un port de sortie et le timer interne du processeur) (figure 8).
Le principe de fonctionnement est le suivant :

On charge depuis l'ordinateur (PC) un algorithme vers le microprocesseur via une carte d'interface et le circuit allume une LED (bit à 0 sur le port P1 out) en cas d'asynchronisme de décharge.

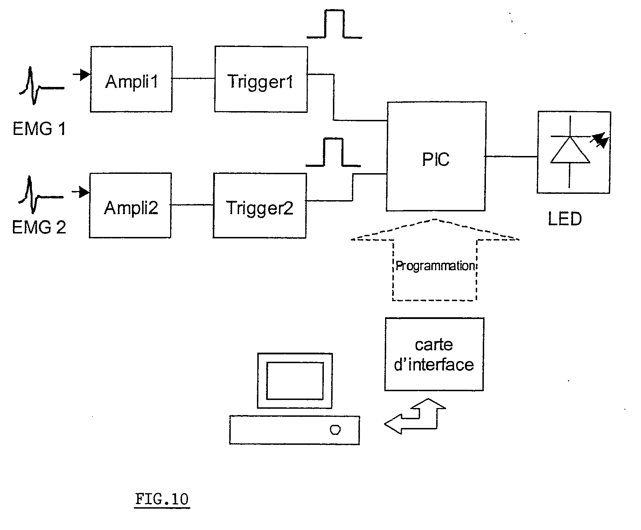

En outre, un algorithme peut analyser si le déphasage entre deux impulsions reste plus ou moins constant (à une certaine gigue près). A chaque itération, on compare le délai entre deux impulsions au délai précédemment mesuré.
Si ce délai reste plus ou moins constant, alors les deux fibres sont innervées par le même motoneurone, sinon, la LED s'allume (figures 9 à 10).

Le dispositif de l'invention peut présenter différentes configurations ergonomiques avec des éléments de mesure et/ou un écran affichant les mesures de fréquence sur différents objets, tels qu'une montre, un gant, un pendentif, un tableau de bord d'un véhicule ou un marteau testeur de réflexes. L'invention peut être également incorporée dans d'autres éléments permettant de diagnostiquer et d'assurer le suivi de tremblements par le patient lui-même ou par le personnel médical.

Le dispositif de l'invention tient dans une main, ce qui permet à l'examinateur de détecter des tremblements psychogènes.

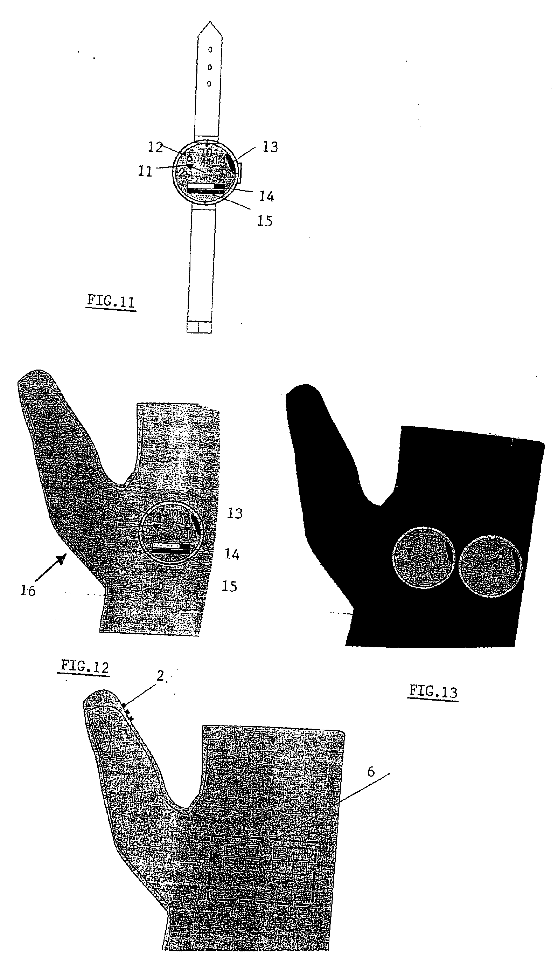

Le dispositif (placé dans la main gauche de l'examinateur) enregistre l'activité rythmique du muscle biceps droit d'un patient qui mime un tremblement du coude droit. L'examinateur utilise sa main droite pour donner des stimulations cutanées de la fréquence croissante sur le bras gauche du patient. Le dispositif informe l'examinateur, de manière instantanée sur la fréquence du tremblement et celui-ci observe que la fréquence des contractions du coude droit du patient est hautement variable, ce qui traduit un tremblement psychogène. De plus, l'examinateur peut tourner le dos au patient qui mime le tremblement. Ce patient va alors réduire ou stopper le tremblement (le patient cesse le pseudo-tremblement lorsque personne ne l'observe) mais l'affichage instantanée sur le dispositif (la montre) révèle à l'examinateur que le patient n'a pas de tremblement lorsqu'il lui tourne le dos. Dans ce cas, l'écran (6) est présent sur une montre (fig. 11) qui comprend un indicateur de la fréquence instantanée (aiguille (11) de la montre), une échelle de gradation des fréquences (12), une zone limite correspondant à une fréquence connue (13) telle que la fréquence du tremblement orthostatique, un crest factor (14) pour mesurer la bouffée EMG la plus intense et un indicateur (15) de la fréquence du tremblement orthostatique sur le nycthémère. Si le curseur est totalement à droite, cela correspond à 24 h/24.

Dans une autre variante, il est possible d'incorporer l'écran (6) sur la surface d'un gant (fig. 12 à 14). Lors d'un traumatisme sévère avec hémorragie chez le patient, par exemple lors d'un accident de roulage, le patient peut présenter un tremblement sévère. L'examinateur (ambulancier, infirmier, urgentiste ...) va manipuler le patient avec le gant (16) afin de réduire les risques de transmission d'infections mais également afin de pouvoir suivre le tremblement et sa réponse au traitement, tout en assurant les soins au patient (les doigts dans le gant sont toujours mobiles). Les différents éléments présents sur l'écran sont similaires à ceux présents sur une montre. Il est également possible de prévoir une version à plusieurs canaux afin de comparer la fréquence de tremblement dans plusieurs parties de membres ou des membres différents.

En outre, le dispositif de l'invention peut comporter des électrodes (2) situés face à l'extrémité (pulpe) des doigts (1 amplificateur actif par doigt, soit 5 pour une main), l'écran (6) étant situé sur le dos du gant (16). Des systèmes de connexion peuvent être également incorporés dans le gant ou sur le gant (de préférence intégrés dans le textile). L'utilisation d'un gant (16) par une analyse multi-canaux permet de tirer profit de la dextérité de l'examinateur qui peut palper le muscle et orienter les doigts selon les zones les plus actives. Cet acte de palpation est un acte très important en étude clinique et oriente fortement le diagnostic neurologique.

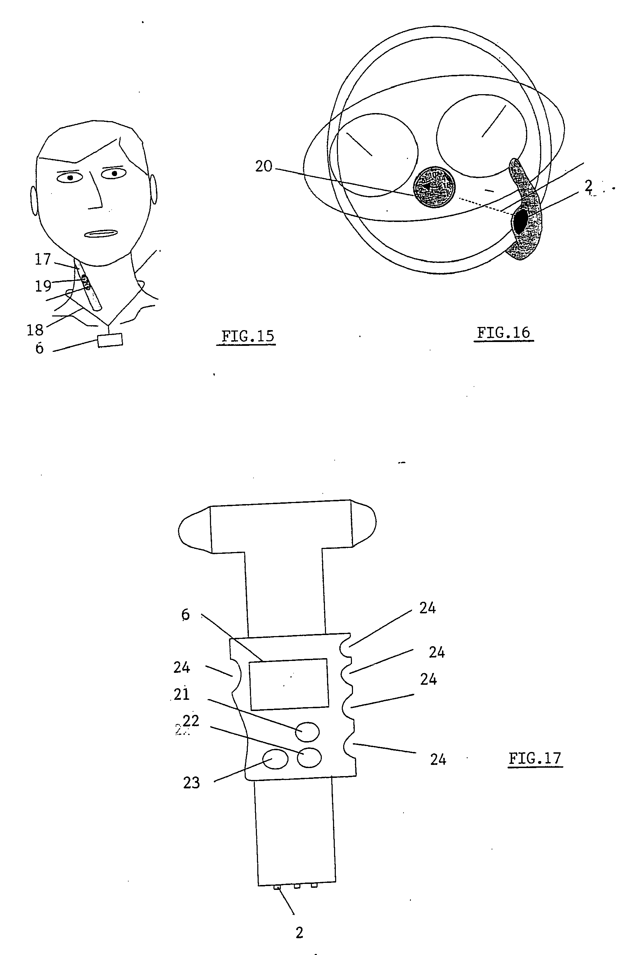

La fig. 15 représente l'utilisation d'un pendentif (18) permettant d'analyser le tremblement des muscles du cou ; car celui-ci peut se dissimuler sous un tee-shirt et le patient peut regarder lui-même la fréquence du tremblement. Lorsque le tremblement est de fréquence très variable, le patient sait que la réponse au traitement médicamenteux sera faible et que, par exemple, il devra éviter de sortir en public durant cette période. Le dispositif de l'invention est donc fixé directement sur le muscle sterno-clédio-mastoidien (17), l'écran affichant (6) la fréquence est incorporé sur le pendentif (18). La communication entre l'électrode active (19) et l'écran affichant la fréquence sur le pendentif (18) peut être effectuée par des fils qui sont dissimulés dans le collier du pendentif. L'écran (6) peut être également incorporé sur le tableau de bord (20) d'un véhicule. En effet, une fatigue musculaire peut générer un tremblement qui peut ainsi être un signe pour le conducteur qu'il doit s'arrêter ou changer la position de sa main (fig. 16) comprenant l'électrode (2).

Tel que représenté à la fig. 17, le dispositif de l'invention peut être également placé dans la poche d'un tablier de l'examinateur et présente une configuration adaptée pour une prise manuelle comme celle d'un marteau à réflexes très utilisé en neurologie. En déposant le manche du marteau sur le muscle étudié, on identifie la fréquence du tremblement s'affichant de manière instantanée sur l'écran STM. Le marteau (25) dispose donc d'encoches (24) épousant la forme des doigts pour une meilleure prise du marteau. Le dispositif comportera simplement des dispositifs supplémentaires, tels que des boutons affichant le menu (21), des boutons on/off (22), des boutons pour figer une courbe particulière d'intérêt (23) et des emplacements pour les doigts (24). Dans ce cas, le dispositif comprend également des électrodes actives (2). Cependant, le marteau à réflexes peut également comporter des électrodes implantées sur le muscle du patient et une communication sans fils peut se faire entre les électrodes et le marteau à réflexes (25) comprenant l'écran d'affichage (6). On dispose ainsi d'un dispositif avec auto-ajustement du gain de l'amplificateur actif, ce qui permet de détecter des tremblements dont les bouffées sont caractérisées par des décharges de petite amplitude.

## Revendications

1. Dispositif portatif de mesure des fréquences de tremblements musculaires d'un sujet, comportant des électrodes de mesure dites actives (2) aptes à pénétrer la peau et/ou le muscle du sujet, connectés à un micro-contrôleur (5) collectant les courants électriques mesurés par les électrodes et affichant instantanément la fréquence correspondante dudit tremblement du sujet sur un écran (6).

2. Dispositif selon la revendication 1, **caractérisé en ce que** le micro-contrôleur est un micro-contrôleur de type PIC.

3. Dispositif selon la revendication 1 ou 2, **caractérisé en ce que** les électrodes de mesure dites actives (2) comportent des filtres intégrés pour réduire ou éliminer les bruits de fond.

4. Dispositif selon les revendications 1 ou 3, **caractérisé en ce qu'**il comporte un système d'alimentation autonome, tel qu'une pile (7).

5. Dispositif selon l'une quelconque des revendications précédentes 1 à 4, **caractérisé en ce que** les électrodes effectuent une mesure de courants électriques correspondant à des fréquences des tremblements du sujet comprises entre 0,01 Hz et 50 Hz, de préférence comprises entre 1 Hz et 25 Hz.

6. Dispositif selon la revendication 5, **caractérisé en ce que** la fréquence mesure est la fréquence du tremblement orthostatique, comprise entre 13 Hz et 17 Hz.

7. Dispositif selon l'une quelconque des revendications précédentes, **caractérisé en ce que** le micro-contrôleur (5) est connecté à des micro-électrodes d'enregistrement de l'activité de muscles (8) d'un sujet et des micro-électrodes de stimulation de nerfs (9) d'un sujet liées à un motoneurone.

8. Dispositif selon la revendication 7, **caractérisé en ce que** le micro-contrôleur (5) effectue un traitement d'un signal FFT et d'un signal TTL de mesure et une comparaison avec des mesures des micro-électrodes d'enregistrement (2) de l'activité de muscles (8) et des micro-électrodes de stimulation de nerfs (9) liées à un motoneurone et une conversion de l'analyse du signal par l'intermédiaire d'une LED.

9. Dispositif selon l'une quelconque des revendications précédentes, **caractérisé en ce que** le sujet est un patient humain.

10. Dispositif selon l'une quelconque des revendications précédentes, **caractérisé en ce que** le tremblement musculaire est un tremblement des muscles d'un membre ou des muscles du cou et/ou de la tête du sujet.

11. Dispositif selon l'une quelconque des revendications précédentes, **caractérisé en ce qu'**au moins l'écran (6) du dispositif est intégré dans un objet choisi parmi le groupe constitué par une montre, un gant (16), un pendentif (18), un tableau de bord (20) d'un véhicule ou un marteau testeur de réflexes (25).
